(19) Europäisches Patentamt
European Patent Office
Office européen des brevets

(11) Publication number: 0 246 774
A1

(12) # EUROPEAN PATENT APPLICATION

(21) Application number: 87303915.0

(22) Date of filing: 30.04.87

(51) Int.Cl.[3]: C 07 D 401/12
C 07 D 491/04, A 61 K 31/41-5
A 61 K 31/44, C 07 D 213/73

(30) Priority: 30.04.86 GB 8610607

(43) Date of publication of application:
25.11.87 Bulletin 87/48

(84) Designated Contracting States:
AT BE CH DE ES FR GB GR IT LI LU NL SE

(71) Applicant: SMITH KLINE & FRENCH LABORATORIES LIMITED
Mundells
Welwyn Garden City Hertfordshire, AL7 1EY(GB)

(72) Inventor: Ife, Robert John
19 Mobbsbury Way
Stevenage Hertfordshire(GB)

(74) Representative: Giddings, Peter John, Dr. et al,
Smith Kline & French Laboratories Ltd. Corporate Patents
Mundells
Welwyn Garden City Hertfordshire AL7 1EY(GB)

(54) **Chemical compounds.**

(57) Compounds of structure (I)

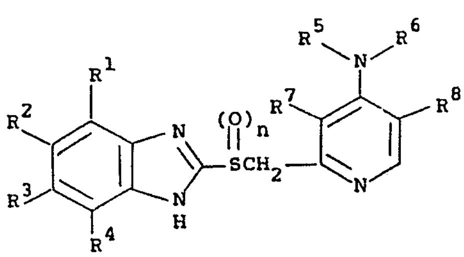

in which, $R^1$ to $R^4$ are the same or different and are each hydrogen, halogen, trifluoromethyl, $C_{1-6}$alkyl, $C_{1-6}$alkoxy, $C_{1-6}$alkanoyl, $C_{1-6}$alkoxycarbonyl, $RCF_2O$, an ethoxy group substituted by 3 to 5 fluorine atoms, or $R^2$ and $R^3$ together form a group $-O(CR_2)_mO-$; R is hydrogen or fluorine; m is 1 or 2; n is 0 or 1; $R^5$ and $R^6$ are the same or different and are each hydrogen, $C_{1-6}$alkyl or $C_{3-6}$cycloalkyl or $R^5$ and $R^6$ together with the nitrogen atom to which they are attached form an azetidino, pyrrolidino, piperidino, piperazino, N-$C_{1-4}$alkylpiperazino or morpholino group; and one of $R^7$ and $R^8$ is fluorine, and the other is hydrogen, fluorine or $C_{1-6}$alkyl; processes and intermediates for their preparation, formulations containing them and their use in therapy for the inhibition of exogenously and endogenously stimulated gastric acid secretion.

EP 0 246 774 A1

# Chemical Compounds

The present invention relates to novel substituted benzimidazole derivatives, processes for their preparation, intermediates useful in their preparation, pharmaceutical compositions containing them and their use in therapy.

Substituted benzimidazole derivatives that are capable of inhibiting gastric acid secretion are known in the art, in for example, EP 5129B, EP 80602A, and GB 2134523A. Such compounds are believed to exert their effects by inhibition of the gastro-intestinal $H^+$-$K^+$ ATPase enzyme (Fellenius E., Berglindh, T., Sachs, G., Olke, L., Elander, B., Sjostrand, S.E. and Wallmark B., 1981, Nature, 290, 159-61). In addition, pending EP 184322A discloses a series of 2-pyridylalkylsulphinyl- and 2-pyridylalkylthio-benzimidazoles in which the pyridyl group is substituted in the 4-position by an optionally substituted amino group, and in the 3- and/or 5-position by a halogen group.

The present invention provides a novel sub-class of compounds falling within the scope of EP 184322-A in which the pyridyl group is substituted in the 3- and/or 5-positions by a fluorine atom.

The present invention therefore provides in a first aspect compounds of structure (I) :

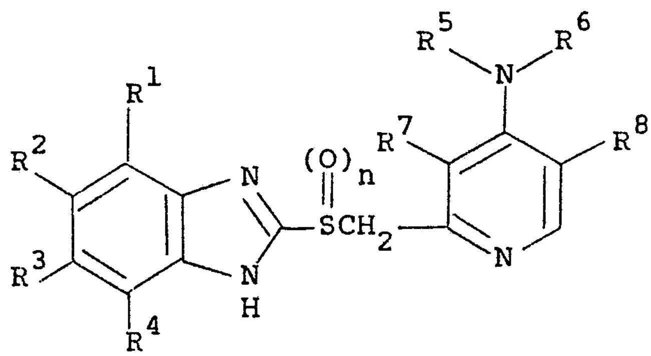

(I)

in which,

$R^1$ to $R^4$ are the same or different and are each hydrogen, halogen, trifluoromethyl, $C_{1-6}$alkyl, $C_{1-6}$alkoxy, $C_{1-6}$alkanoyl, $C_{1-6}$alkoxycarbonyl, $RCF_2O$, an ethoxy group substituted by 3 to 5 fluorine atoms, or $R^2$ and $R^3$ together form a group $-O(CR_2)_mO-$;

R is hydrogen or fluorine;

m is 1 or 2;

n is 0 or 1;

$R^5$ and $R^6$ are the same or different and are each hydrogen, $C_{1-6}$alkyl or $C_{3-6}$cycloalkyl or $R^5$ and $R^6$ together with the nitrogen atom to which they are attached form an azetidino, pyrrolidino, piperidino, piperazino, N-$C_{1-4}$alkylpiperazino or morpholino group; and

one of $R^7$ and $R^8$ is fluorine, and the other is hydrogen, fluorine or $C_{1-6}$alkyl;

or a pharmaceutically acceptable salt thereof.

Suitably, $R^1$ to $R^4$ are all hydrogen. Preferably $R^1$ and $R^4$ are hydrogen, one of $R^2$ and $R^3$ is hydrogen, $C_{1-6}$alkyl, or $C_{1-6}$alkoxy and the other is $C_{1-6}$alkyl or $C_{1-6}$alkoxy.

Suitably, ethoxy groups substituted by 3 to 5 fluorine atoms are, 2,2,2-trifluoroethoxy, 1,1,2-trifluoroethoxy, 1,2,2-trifluoroethoxy, 1,2,2,2-tetrafluoroethoxy and perfluoroethoxy. Preferably, ethoxy groups substituted by 3 to 5 fluorine atoms are 1,1,2,2-tetrafluoroethoxy.

Groups $-O(CR_2)_mO-$ in which m is 1 are methylenedioxy ($-OCH_2O-$) and difluoromethylenedioxy ($-OCF_2O-$). Groups $-O(CR_2)_mO-$ in which m is 2 are ethylenedioxy ($-OCH_2CH_2O-$) and trifluoroethylenedioxy ($-OCHFCF_2O-$).

Suitably n is 0. Preferably n is 1.

Suitably one of $R^5$ and $R^6$ is hydrogen and the other is $C_{3-6}$cycloalkyl. Preferably $R^5$ and $R^6$ are the same or different and are each hydrogen or $C_{1-6}$alkyl. Most preferably $R^5$ and $R^6$ are the same and are each $C_{1-6}$alkyl.

Suitably $R^5$ and $R^6$ together with the nitrogen to which they are attached form an azetidino group. Preferably, $R^5$ and $R^6$ together with the nitrogen to which they are attached form a morpholino, pyrrolidino, piperazino, N-$C_{1-4}$ alkylpiperazino or a piperidino group.

Suitably $R^7$ and $R^8$ are both fluorine.

Preferably, one of $R^7$ and $R^8$ is fluorine and the other is hydrogen or $C_{1-6}$alkyl.

$C_{1-6}$Alkyl groups alone or as part of another group (for example $C_{1-6}$alkoxy, $C_{1-6}$alkoxycarbonyl or $C_{1-6}$alkanoyl), can be straight or branched, for example methyl, ethyl, n-propyl, i-propyl, i-butyl, s-butyl, n-butyl, n-pentyl, i-pentyl or n-hexyl. Preferably $C_{1-6}$alkyl groups are methyl or ethyl.

Preferably, $C_{1-6}$ alkoxy groups are methoxy or ethoxy.

Preferably $C_{1-6}$alkoxycarbonyl groups are methoxycarbonyl or ethoxycarbonyl.

Preferably $C_{1-6}$alkanoyl groups are methanoyl or ethanoyl.

$C_{3-6}$Cycloalkyl groups are cyclopropyl, cyclobutyl, cyclopentyl and cyclohexyl. Preferably, $C_{3-6}$ cycloalkyl groups are cyclopentyl or cyclohexyl.

Preferred compounds of the present invention include

5-methoxy-2-(4-dimethylamino-5-fluoro-2-pyridylmethyl-sulphinyl)-(1H)-benzimidazole

5-methoxy-2-(4-dimethylamino-3-fluoro-2-pyridylmethyl-sulphinyl)-(1H)-benzimidazole

5-methoxy-2-(4-morpholino-3-fluoro-2-pyridylmethyl-sulphinyl)-(1H)-benzimidazole

4,5-dimethoxy-2-(4-morpholino-3-fluoro-2-pyridylmethyl-sulphinyl)-(1H)-benzimidazole

4,5-dimethoxy-2-(4-dimethylamino-3-fluoro-2-pyridylmethyl-sulphinyl)-(1H)-benzimidazole

5-methoxy-2-(4-morpholino-5-fluoro-2-pyridylmethyl-sulphinyl)-(1H)-benzimidazole

5-methoxy-2-(4-morpholino-3-methyl-5-fluoro-2-pyridylmethyl-sulphinyl)-(1H)-benzimidazole

5-methyl-6-methoxy-2-(4-morpholino-3-fluoro-2-pyridylmethyl sulphinyl)-(1H)-benzimidazole

Compounds of structure (I) in which n is O, can form pharmaceutically acceptable acid addition salts with suitable organic and inorganic acids, the nature of which will be apparent to persons skilled in the art. For example, pharmaceutically acceptable salts can be formed by reaction with hydrochloric, sulphuric, sulphonic or phosphonic acids; aliphatic, alicyclic, aromatic or heterocyclic carboxyl or sulphonic acids; methionine, tryptophan, lysine or arginine and the like.

Compounds of structure (I) in which n is 1 can also form pharmaceutically acceptable acid addition salts, but in aqueous solution the salts are less stable than those formed with the compounds of structure (I) in which n is 0.

Compounds of structure (I) in which n is 1 can form basic salts by reaction with an appropriate base. Such salts include, for example, the sodium , potassium, lithium, calcium and magnesium salts which can be prepared by methods well known to those skilled in the art for example, the sodium, potassium and lithium salts can be prepared by reaction with sodium, potassium or lithium hydroxide in an aqueous or non-aqueous medium; and the calcium salts can be prepared by reaction with calcium chloride in an aqueous or non-aqueous medium.

Compounds of structure (I) in which n is 0 can also form basic salts but such salts are less stable than those prepared from compounds of structure (I) in which n is 1.

Compounds of structure (I) in which n is 1 have an asymmetric centre at the S atom and are thus optically active compounds. As such, these compounds exist as two optical isomers (enantiomers). In addition, compounds of structure (I) in which one or more of $R^1$ to $R^8$ is a branched $C_{3-6}$alkyl group (either alone or as part of another group) may contain an additional asymmetric centre(s) due to the presence of the $C_{3-6}$alkyl group(s). Again, such compounds will exist as two (or more) optical isomers.

Both the pure enantiomers, racemic mixtures (50% of each enantiomer) and unequal mixtures of the two are included within the scope of the present invention. Further, all diasteriomeric forms possible (pure enantiomers and mixtures thereof) are within the scope of the invention.

It should be noted that for all the compounds of the present invention, the substituents $R^1$ and $R^4$ as well as $R^2$ and $R^3$ are considered to be equivalent at room temperature in solution. This is due to the tautomerism of the benzimidazole nucleus causing an equilibrium between the 2 possible forms.

In a further aspect the present invention provides a process for the preparation of a compound of structure (I) or a pharmaceutically acceptable salt thereof which comprises

a) reacting a compound of structure (II)

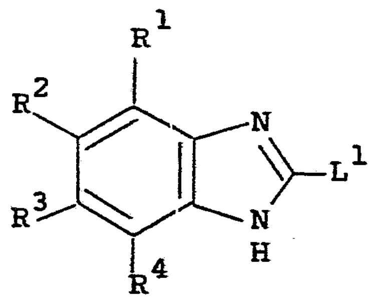

(II)

with a compound of structure (III)

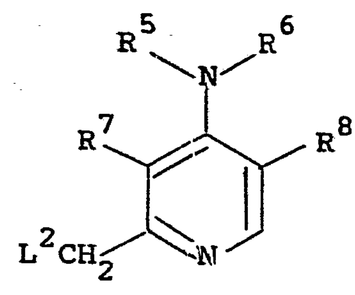

(III)

in which $R^1$ to $R^8$ are as described for structure (I) and one of $L^1$ and $L^2$ is SH and the other a leaving group displaceable by a mercaptan;

(b) reacting a compound of structure (IV)

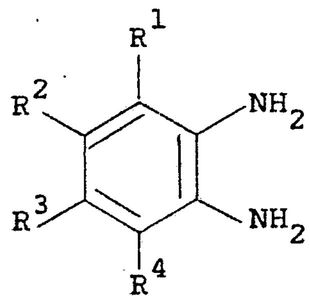

(IV)

in which $R^1$ to $R^4$ are as described for structure (I), with a compound of structure (V)

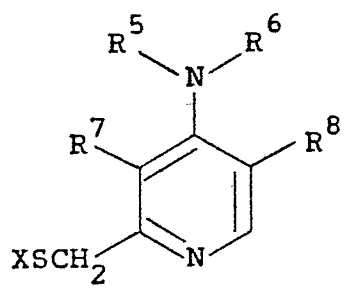

(V)

in which $R^5$ to $R^8$ are as described for structure (I), X is $CO_2H$ or $CSX^1$ and $X^1$ is halogen or $C_{1-4}$alkoxy;

(c) reacting a compound of structure (VI)

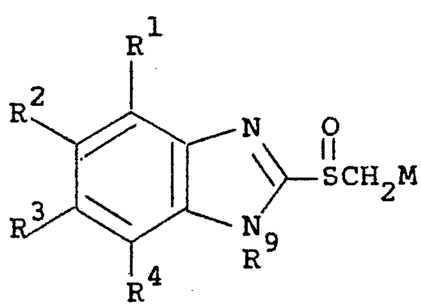

(VI)

in which $R^1$ to $R^4$ are as described for structure (I), $R^9$ is hydrogen or a protecting group and M is an alkali metal atom, with a compound of structure (VII)

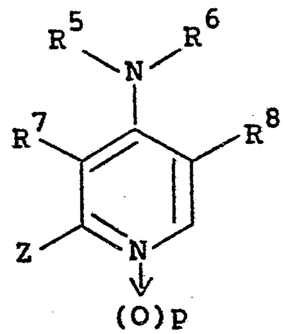

(VII)

in which $R^5$ to $R^8$ are as described for structure (I), Z is a leaving group and p is 0 or 1;

(d) reacting a compound of structure (VIII)

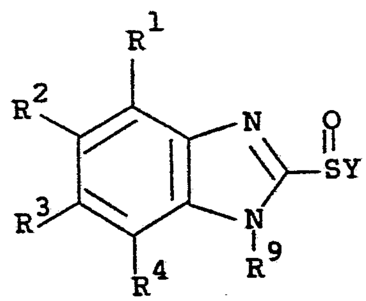

(VIII)

in which $R^1$ to $R^4$ are as described for structure (I), $R^9$ is hydrogen or a protecting group and Y is a leaving group, with a compound of structure (IX)

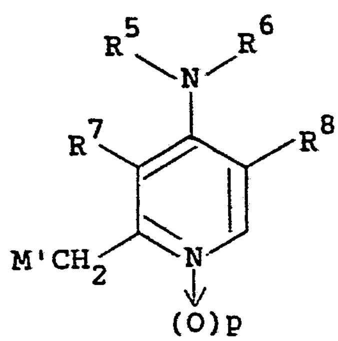

(IX)

in which $R^5$ to $R^8$ are as described for structure (I), p is 0 or 1 and M' is an alkali metal atom or the equivalent of an alkali metal atom.

and, optionally, where desired :

(i) oxidising a compound of structure (I) so formed in which n is O to a compound of structure (I) in which n is 1;

(ii) reducing a compound of structure (I) so formed in which n is 1 to a compound of structure (I) in which n is O;

(iii) forming a pharmaceutically acceptable salt.

Suitable leaving groups $L^1$ displaceable by mercaptan include halogen, for example chloro, bromo or iodo, aryl sulphonyloxy for example toluenesulphonyloxy, alkylsulphonyloxy for example methanesulphonyloxy, alkylmercapto, for example methylmercapto, or alkylsulphinyl, for example methylsulphinyl.

Suitable leaving groups $L^2$ are as described for $L^1$, and may also be $C_{1-4}$acyloxy, for example acetoxy, or hydroxy.

Suitable alkali metal atoms include, for example lithium, sodium or potassium.

Suitable leaving groups Z include, for example, halogen (preferably chloro) and hydroxy activated by esterification with, for example, an aryl or alkane sulphonic acid. Suitable sulphonic acids will be apparent to those skilled in the art, for example p-toluenesulphonic acid or methanesulphonic acid.

Suitable leaving groups Y are those groups which form a reactive sulphinic acid derivative together with the sulphinyl group to which it is attached, and include for example, $C_{1-4}$alkoxy, di-$C_{1-4}$alkylamino and $C_{1-4}$alkyl-mercapto.

Suitable groups M' which are equivalent to a metal atom include, for example, alkali earth metal atoms, (for example magnesium) which are substituted by a halogen atom (for example, bromine).

Suitable protecting groups $R^9$ are those conventional in the art for example as described in "Protective Groups in Organic Synthesis" T.W. Greene 1981 (Wiley). It will be appreciated that the group $R^9$ should not be cleavable under the conditions of reaction of compounds of structure (VIII) and (IX). Such groups include for example benzyl or trityl groups.

The reaction between compounds of structure (II) in which $L^1$ is SH and compounds of structure (III) in which $L^2$ is a leaving group can be carried out under basic conditions in the presence of an inert solvent at a temperature between ambient and the reflux temperature of the solvent.

Suitable solvents include lower alkanols, for example methanol or ethanol, mixtures of lower alkanols with water, or ethers for example dimethoxyethane or tehrahydrofuran.

Suitable bases will be apparent to those skilled in the art and include for example, alkali metal hydroxides, for example, sodium or potassium hydroxide, alkali metal alkoxides, for example potassium t-butoxide, alkali metal hydrides, for example sodium or potassium hydride, or organic tertiary amines, for example triethylamine.

Preferably the reaction is carried out at ambient temperature in ethanol as solvent, in the presence of sodium hydroxide solution.

It is to be noted, and will be apparent to persons skilled in the art that under basic conditions $L^2$ should be a group other than hydroxy or acetoxy, for example halogen, preferably chlorine.

Further, the reaction can be carried out under neutral conditions in the presence of an inert solvent at the reflux temperature of the solvent. Suitable solvents include those hereinbefore described.

Alternatively, when $L^2$ is hydroxy or $C_{1-4}$acyloxy, for example acetoxy, the reaction can be carried out under acidic conditions. Suitable acidic conditions will be well known to those skilled in the art, for example, under reflux in hydrobromic acid, optionally in the presence of acetic acid.

The reaction between compounds of structure (II) in which $L^1$ is a leaving group and compounds of structure (III) in which $L^2$ is SH can be carried out under basic conditions as described for the reaction between compounds of structure (II) in which $L^1$ is SH and compounds of structure (III) in which $L^2$ is a leaving group.

The reaction between compounds of structure (IV) and compounds of structure (V) can be carried out under acidic conditions in a suitable solvent at a temperature between ambient and reflux temperature of the solvent used.

Suitably the reaction is carried out in polar solvents, for example, lower alkanols, dimethyl sulphoxide, acetone, dimethylformamide or acetonitrile, optionally in the presence of water. Preferably the reaction is carried out in ethanol.

Suitably the reaction is carried out in the presence of a strong acid, for example hydrobromic or hydrochloric acid. Preferably the reaction is carried out in the presence of hydrochloric acid.

Preferably the reaction is carried out at the reflux temperature of the solvent.

The reaction between a compound of structure (VI) and a compound of structure (VII) can be carried out in an inert solvent at ambient or elevated temperature depending on the nature of groups M and Z. Suitable solvents include those solvents usually employed for the reaction of enolate ions with alkylating agents for example, benzene or toluene. Preferably, when M is lithium and Z is chlorine, the reaction is conducted in benzene at reflux temperature.

The reaction between a compound of structure (VIII) and a compound of structure (IX) can be carried out under conditions normally used for organometallic reactions as will be well known to those skilled in the art.

The products of reactions (a) to (c) are compounds of structure (I) in which n is 0. These products can be oxidised to compounds of structure (I) in which n is 1 by reaction with an oxidising agent. Suitable oxidising agents include, for example, nitric acid, hydrogen peroxide, peracids, peresters, ozone, dinitrogen tetroxide, iodosobenzene, N-halosuccinamide, 1-chlorobenzotriazole, hypohalites, for example sodium hypochlorite or t-butyl

hypochlorite, diazabicylo [2,2,2]-octane bromine complex, sodium metaperiodate, selenium dioxide, manganese dioxide, chromic acid, ceric ammonium nitrate, bromine, chlorine, or sulphuryl chloride. Preferably the oxidising agent is m-chloroperbenzoic acid.

The oxidation reaction is carried out under conditions known in the art for the oxidation of thiols to sulphoxides. Suitably, the reaction is carried out in an inert solvent at a temperature of between -70° and the boiling point of the solvent used. Suitable solvents include aromatic or chlorinated hydrocarbons, for example benzene, toluene, dichloromethane or chloroform, esters, for example ethyl acetate, or ethers, for example dioxan. Preferably, the reaction is carried out in dichloromethane at a temperature of between -50° and +20°C.

The compounds of structure (I) are obtained either as the free base, or in the form of a salt depending on the choice of starting materials and reaction conditions. If the free compound is obtained it can be converted into a salt by standard techniques well-known to those skilled in the art, for example by dissolving the compound in a suitable solvent and adding the desired acid or base; alternatively, if a salt is obtained it can be converted into the free compound, again by standard techniques, for example by treatment with an appropriate acid or base.

Racemic mixtures may be produced and can be separated by standard techniques e.g. recrystallisation from optically active solvent or by high performance liquid affinity chromatography as described by S. Allenmark, B. Bomgren, H. Baren and P-O Lagerstrom in Analytical Biochemistry, 136, 293-7, 1984.

The intermediate of structure (IV) and the intermediate benzimidazole structures (II), (VI) and (VIII) are known and can be prepared by methods analogous to those known in the art. For example, compounds of structure (II) in which $L^1$ is SH can be prepared by reacting the corresponding compounds of structure (IV) with carbon disulphide in the presence if alkali metal hydroxides, or with potassium ethylxanthate (Org. Syn., 30, 56) or thiophosgene. Compounds of structure (II) in which $L^1$ is a leaving group, for example halogen can be obtained from the corresponding compounds of structure (II) in which $L^1$ is hydroxy by treatment with for example, phosphorous oxychloride. The compounds of structure (II) in which $L^1$ is hydroxy can be prepared by reacting compounds of structure (IV) with phosgene. Compounds of structure (IV) can be prepared by methods analogous to those described in EP 127763A, DE 2848531, CA, 60, 13352h, 1964 and Liebigs Ann. Chem., 730, 16-30, 1969.

Compounds of structure (VI) can be prepared by methylation, oxidation and subsequent deprotection of compounds of structure (II) in which $L^1$ is SH using, for example, alkali metal hydroxides or alcoholates.

The intermediates of structures (III), (V), (VII) and (IX) are novel and provide a further aspect of the invention. They can be prepared by methods analogous to those well known in the art for example as described in "The Chemistry of Heterocyclic Compounds - Pyridine and its Derivatives", Pts. 2 and 3, E. Klingsberg Ed., Interscience Publishers, 1962. For example,

(i) compounds of structure (III) in which $L^2$ is hydroxy or chloro, $R^7$ is fluoro and $R^8$ is hydrogen can be prepared by the reactions outlined in Scheme 1.

Scheme 1

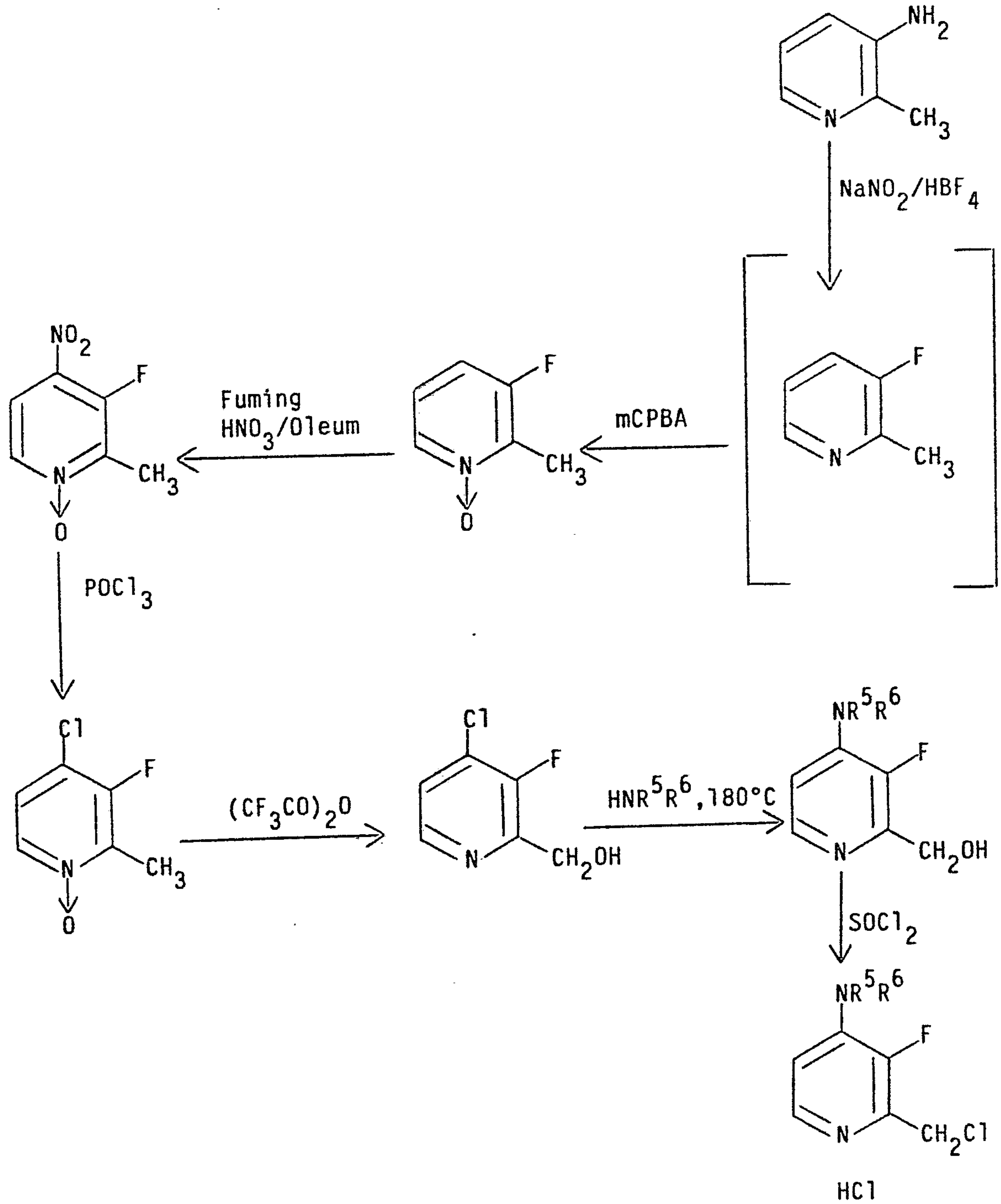


(ii) compounds of structure (III) in which $L^2$ is hydroxy or chloro, $R^7$ is hydrogen, and $R^8$ is fluorine can be prepared by the methods outlined in Scheme 2.

<u>Scheme 2</u>

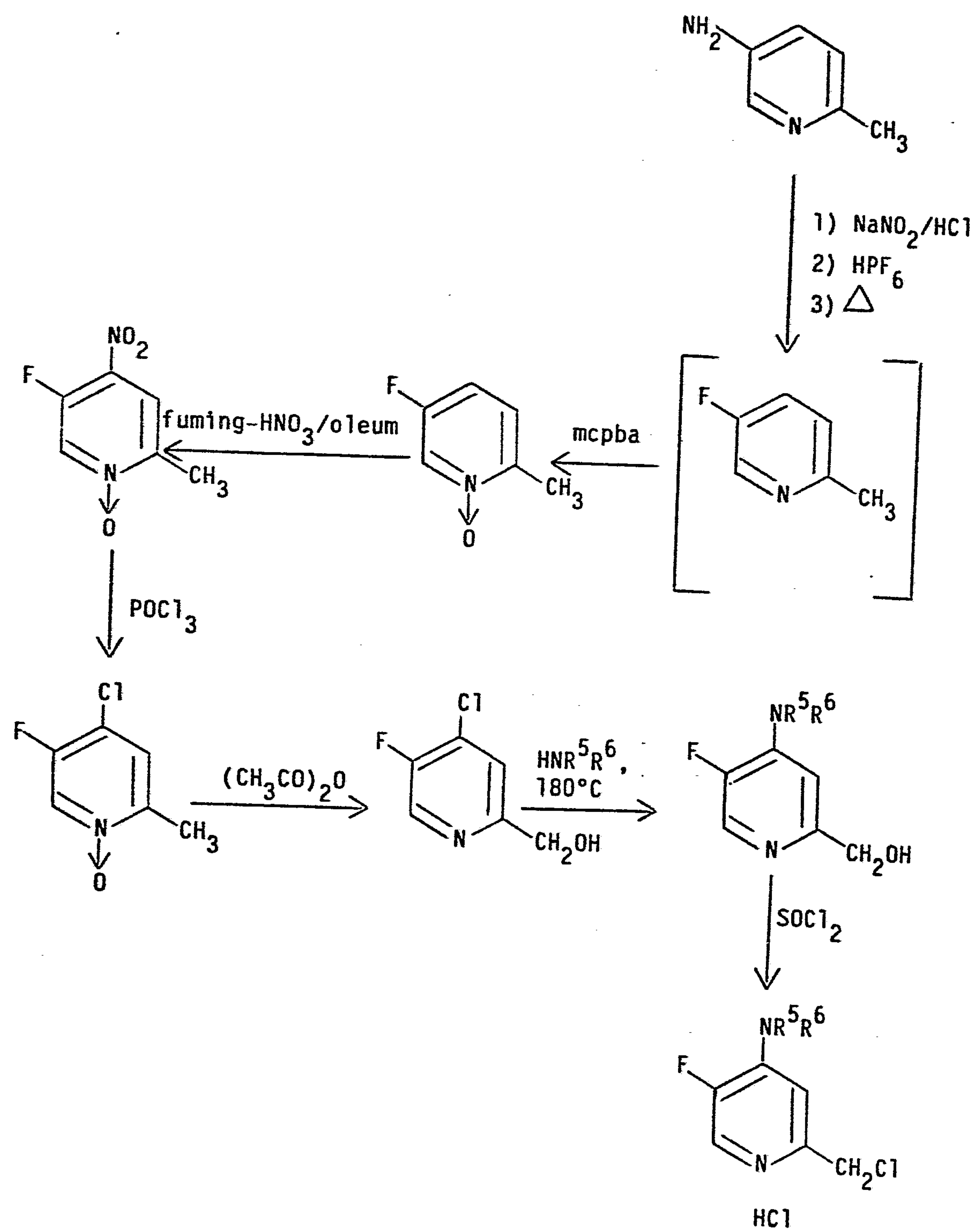
1) $NaNO_2$/HCl
2) $HPF_6$
3) △
mcpba
fuming-$HNO_3$/oleum
$POCl_3$
$(CH_3CO)_2O$
$HNR^5R^6$,
180°C
$SOCl_2$
HCl

In Schemes 1 and 2, the starting 3-amino-2-picoline and corresponding 5-amino-2-picolines can be prepared as described in part 3 of the Klingsberg reference.

Treatment of the compounds of structure (III) in which $L^2$ is OH with for example p-toluenesulphonyl chloride gives the required compounds of structure (III) in which $L^2$ is sulphonyloxy.

Compounds of structure (III) in which $L^2$ is SH can be prepared from compounds of structure (III) in which $L^2$ is a leaving group for example halogen, by reaction with, for example, NaSH.

Compounds of structure (V) can be prepared from the corresponding compounds of structure (III); for example, compounds of structure (V) in which X is CSCl can be prepared by reaction of a compound of structure (III) in which $L^2$ is SH with thiophosgene.

Compounds of structure (VII) can be prepared by methods analogous to those described in Roszniki Chem., 35, 475, 1961.

In a further aspect the present invention provides compounds of structure (I) and pharmaceutically acceptable salts thereof for use in therapy. The compounds of structure (I) and their pharmaceutically acceptable salts inhibit exogenously and endogenously stimulated gastric acid secretion and are useful in the treatment of gastro-intestinal diseases in mammals, in particular humans. Such diseases include, for example, gastric and duodenal ulcers, and Zollinger-Ellison Syndrome.

Further, the compounds of structure (I) can be used in the treatment of other disorders where a cytoprotective

and/or anti-secretory effect is desirable, for example, in patients with gastritis, NSAID induced gastritis, gastritis associated with a history of chronic and excessive alcohol consumption, gastric ulcers, acute upper gastrointestinal bleeding, for the prophylaxis of upper gastrointestinal haemmorage in patients at risk of the development of stress-related lesions of the gastric mucosa, and in the reduction of risk factors, for example gastric acidity and volume associated with pulmonary aspiration.

It is believed that, after administration to mammals, compounds of structure (I) in which n is 0 exert their anti-secretory and cytoprotective activities after conversion into compounds of structure (I) in which n is 1.

Furthermore it is believed that compounds of structure (I) in which n is 1, after administration to mammals, exert their anti-secretory activity after transformation under acid conditions into another chemically reactive species. Active species so generated from compounds of structure (I) are within the scope of the present invention.

In therapeutic use, the compounds of the present invention are usually administered in a standard pharmaceutical composition. The present invention therefore provides in a further aspect pharmaceutical compositions comprising a compound of structure (I) or a pharmaceutically acceptable salt thereof and a pharmaceutically acceptable carrier.

Compounds of structure (I) and their pharmaceutically acceptable salts can be administered in standard manner for example, orally, parenterally, rectally, transdermally, via inhalation or via buccal administration.

The compounds of structure (I) and their pharmaceutically acceptable salts which are active when given orally can be formulated as liquids, for example syrups, suspensions or emulsions, tablets, capsules and lozenges.

A liquid formulation will generally consist of a suspension or solution of the compound or pharmaceutically acceptable salt in a suitable liquid carrier(s) for example, ethanol, glycerine, non-aqueous solvent, for example polyethylene glycol, oils, or water with a suspending agent, preservative, flavouring or colouring agent.

A composition in the form of a tablet can be prepared using any suitable pharmaceutical carrier(s) routinely used for preparing solid formulations. Examples of such carriers include magnesium stearate, starch, lactose, sucrose and cellulose.

A composition in the form of a capsule can be prepared using routine encapsulation procedures. For example, pellets containing the active ingredient can be prepared using standard carriers and then filled into a hard gelatin capsule; alternatively, a dispersion or suspension can be prepared using any suitable pharmaceutical carrier(s), for example aqueous gums, celluloses, silicates or oils and the dispersion or suspension then filled into a soft gelatin capsule.

Compounds of structure (I) in which n is 1 are susceptible to decomposition in acid media, and thus tablets and capsules containing such compounds are preferably provided with an enteric coating to protect the compound from acid degradation in the stomach or capsules used which are inherently acid resistant. Alternatively,

the enteric coating can be provided by coating pellets containing the active ingredient before filling them into the hard gelatin capsule. Suitable enteric coating materials are those well known in the art of pharmacy and include for example shellac or anionic-film forming polymers such as cellulose acetate phthalate and hydroxypropylmethyl cellulose phthalate and the like, optionally in the presence of a plasticizer.

It will be apparent to those skilled in the art that other standard techniques for enhancing the stability of such compounds can be used. The nature of such techniques will depend on the route of administration and include, for example, the formation of stable complexes with, for example ß-cyclodextrin.

The compounds of structure (I) and their pharmaceutically acceptable salts which are active when administered parenterally (i.e. by injection or infusion) can be formulated as solutions or suspensions.

A composition for parenteral administration will generally consist of a solution or suspension of the active ingredient in a sterile aqueous carrier or parenterally acceptable oil, for example polyethylene glycol, polyvinyl pyrrolidone, lecithin, arachis oil or sesame oil. Alternatively, the solution can be lyophilised and then reconstituted with a suitable solvent just prior to administration.

A typical suppository composition comprises a compound of formula (I) or a pharmaceutically acceptable salt thereof which is active when administered in this way, with a binding and/or lubricating agent such as polymeric glycols, gelatins or cocoa butter or other low melting vegetable or synthetic waxes or fats.

A typical transdermal formulation comprises a conventional aqueous or non-aqueous vehicle, for example, a cream, ointment lotion or paste or in the form of a medicated plaster, patch or membrane.

A typical composition for inhalation comprises a solution, suspension or emulsion that may be administered in the form of an aerosol using a conventional propellant such as dichlorodifluoromethane or trichlorofluoromethane.

Preferably the composition is in unit dose form. Each dosage unit for oral administration contains preferably from 1 to 250 mg (and for parenteral administration contains preferably from 1 to 150 mg) of a compound of the formula (I) or a pharmaceutically acceptable salt thereof calculated as the free base.

The compounds of the present invention will normally be administered to a subject for the treatment of gastro-intestinal diseases and other conditions caused or exacerbated by gastric acidity. The daily dosage regimen for an adult patient may be, for example, an oral dose of between 1 mg and 500 mg, preferably between 1 mg and 250 mg, or an intravenous, subcutaneous, or intramuscular dose of between 1 mg and 500 mg, preferably between 1 mg and 150 mg, of the compound of the formula (I) or a pharmaceutically acceptable salt thereof calculated as the free base, the compound being administered 1 to 4 times per day. Suitably the compounds will be administered for a period of continuous therapy, for example for a week or more.

In addition, the compounds of the present invention can be co-administered with further active ingredients, such as antacids (for example magnesium carbonate or hydroxide and aluminium hydroxide), non-steroidal

anti-flammatory drugs (for example indomethacin, aspirin or naproxen), steroids, or nitrite scavengers (for example ascorbic acid or aminosulphonic acid), or other drugs used for treating gastric ulcers, for example pirenzipine, prostanoids for example 16,16 dimethyl $PGE_2$ or histamine $H_2$-antagonists, for example cimetidine.

The following examples illustrate the invention. Temperatures are recorded in degrees centigrade.

## Example 1

## Preparation of 5-Methoxy-2-(4-morpholino-3-fluoro-2-pyridylmethylthio)-(1H)-benzimidazole

(i) Sodium nitrite (15.3 g) was added to a stirred solution of 3-amino-2-picoline (20 g) in 40% fluoroboric acid (225 ml) at -5 to -10°. The mixture was stirred for 2 hours at -10° followed by a further 2 hours at room temperature, after which time, the solution was basified (NaOH) and extracted with dichloromethane. The extracts were back-washed with water (pH 6 HCl) to remove some unreacted amine and, after drying ($K_2CO_3$), m-chloroperbenzoic acid (35.7 g) was added and the mixture allowed to stand for 16 hours. Ammonia gas was passed through the solution and the precipitated benzoate salts filtered off. The filtrate was evaporated to dryness to give 2-methyl-3-fluoropyridine-N-oxide (13.36 g) m.p. softens 55° melts 80°.

(ii) A nitration mixture of 30% oleum (55 ml) and fuming nitric acid (90 ml) was added dropwise with cooling and stirring to a solution of 2-methyl-3-fluoropyridine-N-oxide (13.3 g) in concentrated sulphuric acid (48 ml) at 10-15°. The solution was stirred at room temperature for 1.5 hours and then for 2 hours at 100°. After cooling, the mixture was poured onto ice, basified (ammonium carbonate) and extracted with dichloromethane. After drying ($K_2CO_3$), the extracts were evaporated to dryness to give 2-methyl-3-fluoro-4-nitropyridine-N-oxide (12.12 g) m.p. 170-175°.

(iii) Phosphoryl chloride (19 ml) in dichloromethane (50 ml) was added dropwise to a stirred solution of 2-methyl-3-fluoro-4-nitropyridine-N-oxide (12 g) in dichloromethane (50 ml). The reaction mixture was heated under reflux for a total of 8 hours. After cooling and

pouring onto ice, the mixture was basified (NaOH) to pH 9 and extracted with dichloromethane. After drying ($K_2CO_3$) and removal of solvent, the crude product was purified by column chromatography (silica gel; 1% MeOH/$CHCl_3$) to give 2-methyl-3-fluoro-4-chloro-pyridine-N-oxide (6.78 g) m.p. 63-65°.

(iv) Trifluoroacetic anhydride (16.18 ml) was added dropwise at 10-15° to a solution of 2-methyl-3-fluoro-4-chloropyridine-N-oxide (6.17 g) in dichloromethane (50 ml). After three days at room temperature, methanol (30 ml) was added to the cooled solution. The excess solvent was removed and the residue dissolved in water. After basifying (NaOH), the solution was extracted with dichoromethane. The extracts were dried ($K_2CO_3$) and evaporated to dryness to give a solid which was purified by column chromatography (silica gel; 1% MeOH/$CHCl_3$) to give 2-hydroxymethyl-3-fluoro-4-chloropyridine (3.8 g) m.p. indeterminate)

(v) A mixture of 2-hydroxymethyl-3-fluoro-4-chloro-pyridine (2.5 g) and morpholine (6.74 ml) were heated together in a sealed vessel at 180° for 4 hours. The mixture was cooled, washed out with ethanol and stripped. The residue was treated with water (80 ml) and extracted with chloroform. The combined extracts were dried ($K_2CO_3$) filtered and excess solvent removed to give a solid which, after chromatography (silica gel 1-2% MeOH/$CHCl_3$), afforded 2-hydroxymethyl-3-fluoro-4-morpholinopyridine (1.75 g) m.p. 138-142°.

(vi) Thionyl chloride (1.8 ml) in chloroform (15 ml, alumina dried) was added dropwise to a stirred and cooled solution of 2-hydroxymethyl-3-fluoro-4-morpholinopyridine (1.74 g) in chloroform (20 ml). After 2 hours at room temperature the solution was reduced in volume and ether

added. The precipitate was filtered off, washed and dried to give 2-chloromethyl-3-fluoro-4-morpholinopyridine hydrochloride (2.1 g) m.p. 203-205°.

(vii) 5N NaOH (3.39 ml) was added dropwise to a stirred solution of 2-chloromethyl-3-fluoro-4-morpholinopyridine hydrochloride (2.06 g) and 5-methoxy-(1H)-benzimidazole-2-thiol (1.39 g) in ethanol (35 ml). After 3 hours excess solvent was removed and the residue treated with water (80 ml) and extracted with dichloromethane. The extracts were washed with water, dried ($K_2CO_3$), excess solvent removed and the residue crystallised from ethyl acetate to yield 5-methoxy-2-(4-morpholino-3-fluoro-2-pyridylmethylthio)-(1H)-benzimidazole (2.16 g) m.p. 121-123°.

## Example 2

Preparation of 5-methoxy-2-(4-morpholino-3-fluoro-2-pyridylmethylsulphinyl)-(1H)-benzimidazole

m-Chloroperbenzoic acid (1.06 g) in dichloromethane (50 ml) was added dropwise to a stirred solution of 5-methoxy-2-(4-morpholino-3-fluoro-2-pyridylmethylthio)-(1H)-benzimidazole (2.31 g) in dichloromethane (50 ml) at -30 to -35°. After 0.5 hour at -30° a further addition of m-chloroperbenzoic acid (0.106 g) in dichloromethane (15 ml) was made and the mixture stirred for a further 20 minutes at -30°. Ammonia gas was then passed through the solution and the precipitated benzoate salts filtered off. The filtrate was evaporated to dryness and the residue crystallised from ethyl acetate to yield 5-methoxy-2-(4-morpholino-3-fluoro-2-pyridylmethylsulphinyl)-(1H)-benzimidazole (1.75 g) m.p. 142-46°

$C_{18}H_{19}FN_4O_3S$

Found C,55.46 H,4.94 N,14.27 S,8.08

Requires C,55.37 H,4.91 N,14.35 S,8.21

Example 3

Preparation of 5-methoxy-2-(4-morpholino-5-fluoro-2-pyridylmethylthio)-(1H)-benzimidazole

(i) Sodium nitrite (43.65 g) was added portionwise to a stirred soluton of 5-amino-2-picoline (34.21 g) in water (220 ml) and concentrated hydrochloric acid (158 ml) at -5 to 0° over 30 minutes. After a further 5 minutes at this temperature, 65% hexafluorophosphoric (169 ml) was added dropwise causing precipitation of the hexafluorophosphate salt which was filtered off, washed with water, ethanol and ether and dried under vacuum (76 g). The salt was then added portionwise with rapid stirring to petroleum ether (800 ml, b.p. 120-160°) at 90-95° over 30 minutes. After a further 5 minutes the mixture was cooled to room temperature, the petrol decanted and extracted with 2N hydrochloric acid (400 ml). The acidic extract and solid residue were combined, extracted with ether, basified (NaOH) and extracted again with dichloromethane. After drying ($K_2CO_3$), m-chloroperbenzoic acid (65.5 g) was added and the mixture allowed to stand for 16 hours. Ammonia gas was passed through the solution and the precipitated benzoate salts filtered off. Excess solvent was removed from the filtrate to give 2-methyl-5-fluoropyridine-N-oxide (30.24 g) as an oil.

(ii) Substituting 2-methyl-5-fluoropyridine-N-oxide (33.89 g) for 2-methyl-3-fluoropyridine-N-oxide and using corresponding molar proportions of the other reagents in the method of Example 1(ii), gave 2-methyl-4-nitro-5-fluoropyridine-N-oxide (33.09 g) m.p. 100-105°.

(iii) Substituting 2-methyl-4-nitro-5-fluoropyridine-N-oxide (32.94 g) for 2-methyl-3-fluoro-4-nitropyridine-N-oxide and using corresponding molar proportions of the other reagents in the method of Example 1(iii), gave

2-methyl-4-chloro-5-fluoropyridine-N-oxide (25.97 g) m.p. 100-102°.

(iv) Substituting 2-methyl-4-chloro-5-fluoro-pyridine-N-oxide (13 g) for 2-methyl-3-fluoro-4-chloro-pyridine-N-oxide and using corresponding molar proportions of the other reagents in the method of Example 1(iv), gave 2-hydroxymethyl-4-chloro-5-fluoropyridine (6.83 g) m.p. 50-52°.

(v) Substituting 2-hydroxymethyl-4-chloro-5-fluoro-pyridine (2.5 g) for 2-hydroxymethyl-3-fluoro-4-chloro-pyridine and using corresponding molar proportions of the other reagents in the method of Example 1(v), gave 2-hydroxymethyl-4-morpholino-5-fluoropyridine (2.08 g) m.p. 134-136° (from acetonitrile).

(vi) Substituting 2-hydroxymethyl-4-morpholino-5-fluoropyridine (1.98 g) for 2-hydroxymethyl-4-morpholino-3-fluoropyridine and using corresponding molar proportions of the other reagents in the method of Example 1(vi), gave 2-chloromethyl-4-morpholino-5-fluoropyridine hydrochloride (2.45 g) m.p. 224-226°.

(vii) Substituting 2-chloromethyl-4-morpholino-5-fluoropyridine hydrochloride (2.4 g) for 2-chloromethyl-4-morpholino-3-fluoropyridine and using corresponding molar proportions of the other reagents in the method of Example 1(vii) gave 5-methoxy-2-(4-morpholino-5-fluoro-2-pyridylmethylthio)-(1H)-benzimidazole (2.84 g) m.p. 142-144° (from ethyl/acetate/ether).

Example 4

Preparation of 5-methoxy-2-(4-morpholino-5-fluoro-2-pyridylmethylsulphinyl)-(1H)-benzimidazole

(i) Substituting 5-methoxy-2-(4-morpholino-5-fluoro-2-pyridylmethylthio)-(1H)-benzimidazole (2.77 g) for 5-methoxy-2-(4-morpholino-3-fluoro-2-pyridylmethylthio)-(1H)-benzimidazole and using corresponding molar proportions of the other reagents in the method of Example 2 gave 5-methoxy-2-(4-morpholino-5-fluoro-2-pyridylmethylsulphinyl)-(1H)-benzimidazole (2.08 g) m.p. 109-111° (from ethyl acetate).

$C_{18}H_{19}FN_4O_3.0.15\ H_2O$

Found C,55.11 H,4.91 N,14.18 S,7.88

Requires C,54.98 H,4.95 N,14.25 S,8.16

Example 5

Preparation of 5-Methoxy-2-(4-dimethylamino-3-fluoro-2-pyridylmethylthio)-(1H)-benzimidazole

(i) A mixture of 2-hydroxymethyl-3-fluoro-4-chloropyridine (2.5 g) and 33% dimethylamine in IMS (17 ml) were heated together in a sealed vessel at 180° for 4 hours. The mixture was cooled, washed out with ethanol and stripped. The residue was treated with water (40 ml) and extracted with chloroform. The combined extracts were dried ($K_2CO_3$), filtered and excess solvent evaporated to give a solid which was purified by column chromatography (silica gel; 2% MeOH/$CHCl_3$) to give, after crystallisation ($CHCl_3$ Petroleum ether b.p. 60-80°), 2-hydroxymethyl-4-dimethylamino-3-fluoropyridine (1.84 g) m.p. 88-90°.

(ii) Substituting 2-hydroxymethyl-4-dimethylamino-3-fluoropyridine (1.76 g) for 2-hydroxymethyl-3-fluoro-4-morpholinopyridine and using corresponding molar proportions of the other reagents in the method of

Example 1(vi), gave 2-chloromethyl-4-dimethylamino-3-fluoropyridine hydrochloride (2.27 g) m.p. 209-212°.

(iii) Substituting 2-chloromethyl-4-dimethylamino-3-fluoropyridine hydrochloride (2.21 g) for 2-chloromethyl-4-morpholino-3-fluoropyridine hydrochloride and using corresponding molar proportions of the other reagents in the method of Example 1(vii), gave 5-methoxy-2-(4-dimethylamino-3-fluoro-2-pyridylmethylthio)-(1H)-benzimidazole (3.26 g) as an oil.

Example 6

Preparation of 5-methoxy-2-(4-dimethylamino-3-fluoro-2-pyridylmethylsulphinyl)-(1H)-benzimidazole

(i) Substituting 5-methoxy-2-(4-dimethylamino-3-fluoro-2-pyridylmethylthio)-(1H)-benzimidazole (3.19 g) for 5-methoxy-2-(4-morpholino-3-fluoro-2-pyridylmethylthio)-(1H)-benzimidazole and using corresponding molar proportions of the other reagents in the Example 2, gave 5-methoxy-2-(4-dimethylamino-3-fluoro-2-pyridylmethylsulphinyl)-(1H)-benzimidazole (2.12 g) m.p. 145-147° (from ethyl acetate).

$C_{16}H_{17}FN_4O_2S$ 0.044 $CH_3COOC_2H_5$

Found C,55.28 H,5.03 N,15.88 S,9.06

Requires C,55.15 H,4.97 N,15.90 S,9.10.

Example 7

Preparation of 5-methoxy-2-(4-dimethylamino-5-fluoro-2-pyridylmethylthio)-(1H)-benzimidazole

(i) Substituting 2-hydroxymethyl-4-chloro-5-fluoropyridine (2.5 g) for 2-hydroxymethyl-3-fluoro-4-chloropyridine and using corresponding molar proportions of the other reagents in the method of Example 5(i) gave

2-hydroxymethyl-4-dimethylamino-5-fluoropyridine (2.18 g) m.p. 80-82°.

(ii) Substituting 2-hydroxymethyl-4-dimethylamino-5-fluoropyridine (2.06 g) for 2-hydroxymethyl-4-morpholino-3-fluoropyridine and using corresponding molar proportions of the other reagents in the method of Example 1(vi), gave 2-chloromethyl-4-dimethylamino-5-fluoropyridine hydrochloride (2.68 g) m.p. 211-212°.

(iii) Substituting 2-chloromethyl-4-dimethylamino-5-fluoropyridine hydrochloride (2.61 g) for 2-chloromethyl-4-morpholino-3-fluoropyridine hydrochloride and using corresponding molar proportions of the other reagents in the method of Example 1(vii), gave after chromatography (silica gel 1% MeOH/$CHCl_3$), 5-methoxy-2-(4-dimethylamino-5-fluoro-2-pyridylmethylthio)-(1H)-benzimidazole (3.86 g) as an oil.

## Example 8

**Preparation of 5-methoxy-2-(4-dimethylamino-5-fluoro-2-pyridylmethylsulphinyl)-(1H)-benzimidazole**

Substituting 5-methoxy-2-(4-dimethylamino-5-fluoro-2-pyridylmethylthio)-(1H)-benzimidazole (3.75 g) for 5-methoxy-2-(4-morpholino-3-fluoro-2-pyridylmethylthio)-(1H)-benzimidazole and using corresponding molar proportions of the other reagents in the method of Example 2, gave 5-methoxy-2-(4-dimethylamino-5-fluoro-2-pyridylmethylsulphinyl)-(1H)-benzimidazole (1.93 g) m.p. 99-102° (from ethyl acetate).

$C_{16}H_{17}FN_4O_2S$

Found C,55.44 H,4.90 N,16.07 S,9.15

Requires C,55.16 H,4.92 N,16.08 S,9.20

Example 9

Preparation of 5-methoxy-2-(4-morpholino-5-fluoro-3-methyl-2-pyridylmethylthio)-(1H)-benzimidazole

(i) Sodium nitrite (5.6 g) was added portionwise to a stirred solution of 5-amino-3-methyl-2-picoline (5 g) in conc. hydrochloric acid (25 ml) and water (30 ml) at -5° to 0° over 15 minutes. After a further 15 minutes at this temperature, 65% hexafluorophosphoric acid (25 ml) was added dropwise causing precipitation of the hexafluorophosphate salt which was filtered off, washed with water, ethanol, and ether and dried under vacuum (12.33 g). The salt was added portionwise, with rapid stirring, to petroleum ether (100 ml, b.p. 120°-160°) at 60° over 20 minutes. After a further 5 minutes the mixture was cooled to room temperature, the petroleum ether layer decanted, and extracted with 2N hydrochloric acid. The acidic and oily residues were combined, extracted with ether, basified (NaOH) and extracted again with dichloromethane. After drying ($K_2CO_3$), m-chloroperbenzoic acid (8.45 g) was added to the dichloromethane solution and the mixture allowed to stand for 16 hours. Ammonia gas was passed through the solution and the precipitated benzoate salts filtered off. The filtrate was evaporated to dryness to give 2,3-dimethyl-5-fluoropyridine-N-oxide (2.66 g) m.p. indeterminate.

(ii) A nitration mixture of 30% oleum (24 ml) and fuming nitric acid (40 ml) was added dropwise with cooling to a solution of 2,3-dimethyl-5-fluoropyridine-N-oxide (6.64 g) in concentrated sulphuric acid (24 ml) at 10-15°. The solution was stirred at room temperature for 1.5 hours and then 2 hours at 80-90°. After cooling, the mixture was poured onto ice, basified (ammonium carbonate) and extracted with dichloromethane. After drying ($K_2CO_3$), the extracts were evaporated to dryness to give

2,3-dimethyl-4-nitro-5-fluoro-pyridine-N-oxide (5.88 g) m.p. 80-83°.

(iii) Phosphoryl chloride (8.6 ml) in dichloromethane (50 ml) was added to a stirred solution of 2,3-dimethyl-4- nitro-5-fluoropyridine (5.8 g) in dichloromethane (100 ml). The reaction mixture was heated under reflux for 4 hours. After cooling and pouring onto ice, the mixture was basified (NaOH) to pH 12 and extracted with dichloromethane. After drying ($K_2CO_3$) and removal of solvent, the crude product was purified by column chromatography (silica gel, 2%MeOH/$CHCl_3$) to give 2,3-dimethyl-4-chloro-5-fluoropyridine-N-oxide (4.11 g) m.p. 137-39°.

(iv) Trifluoroacetic anhydride (9.7 ml) in dichloromethane (20 ml) was added dropwise to a solution of 2,3-dimethyl-4-chloro-5-fluoropyridine-N-oxide (4.0 g) in dichloromethane (35 ml) at 10-15°. After 16 hours at room temperature, the mixture was poured onto ice and stirred for 15 minutes. After basifying (NaOH) to pH 13, the dichloromethane was separted, dried ($K_2CO_3$) and evaporated to dryness to yield 2-hydroxymethyl-3-methyl-4-chloro-5-fluoropyridine which was used without further purification.

(v) A mixture of 2-hydroxy-3-methyl-4-chloro-5-fluoropyridine (4 g) and morpholine (9.8 ml) were heated together in a sealed vessel at 180° for 4 hours. The mixture was cooled, washed out with ethanol and evaporated to dryness. The residue was treated with water (80 ml) and extracted with chloroform. The combined extracts were dried ($K_2CO_3$), filtered and excess solvent removed to give an oil which, after chromatography (silica gel, $CHCl_3$), afforded 2-hydroxymethyl-3-methyl-4-morpholino-5-fluoropyridine (0.78 g) m.p. indeterminate.

(vi) Thionyl chloride (0.73 ml) in chloroform (5 ml, alumina dried) was added dropwise to a stirred and cooled solution of 2-hydroxy-3-methyl-4-morpholino-5-fluoro pyridine (0.75 g) in chloroform (15 ml). After 2 hours at room temperature the solution was reduced in volume and ether added. The precipitate was filtered off, washed and dried to give 2-chloromethyl-3-methyl-4-morpholino-5-fluoropyridine hydrochloride (0.6 g) m.p.148-156°.

(vii) 5N NaOH (1.68ml) was added dropwise to a stirred solution of 2-chloromethyl-3-methyl-4-morpholino-pyridine hydrochloride (1.2 g) and 5-methoxy-(1H)-benzimidazole-2-thiol (0.77 g) in ethanol (20 ml). After 16 hours and adding a further 0.2 eq 5N NaOH, excess solvent was removed and the residue treated with water (50 ml) and extracted with dichloromethane. The extracts were washed with water, dried ($K_2CO_3$), excess solvent removed and the residue crystallised from ethanol/water to yield 5-methoxy-2-(4-morpholino-5-fluoro-3-methyl-2-pyridylmethylthio)-(1H)-benzimidazole (1.34 g) m.p. 84-86°.

## Example 10

## Preparation of 5-methoxy-2-(4-morpholino-5-fluoro-3-methyl-2-pyridylmethylsulphinyl)-(1H)-benzimidazole

(i) m-Chloroperbenzoic acid (0.48 g) in dichloromethane (10 ml) was added dropwise to a stirred solution of 5-methoxy-2-(4-morpholino-5-fluoro-3-methyl-2-pyridylmethylthio)-(1H)-benzimidazole (1.1 g) in dichloromethane (50 ml) at -30 to -35°. After 0.5 hour at -30° a further addition of m-chloroperbenzoic acid (0.048 g) in dichloromethane (2 ml) was made and the mixture stirred for a further 0.5 hour. After a further addition of m-chloroperbenzoic acid (0.048 g) in dichloromethane (2 ml) and after standing for a further

0.5 hour at -30°, ammonia gas was passed through the solution and the precipitated benzoate salts filtered off. The filtrate was evaporated to dryness and the residue crystallised from ethyl acetate to yield 5-methoxy-2-(4-morpholino-5-fluoro-3-methyl-2-pyridylmethylsulphinyl)-(1H)-benzimidazole (0.8 g) m.p. 158-160°.

$C_{19}H_{21}FN_4O_3S$

Found C,56.45 H,5.16 N,13.63 S,7.63

Requires C,56.42 H,5.23 N,13.85 S,7.92

Example 11

Preparation of 5,6-dimethoxy-2-(4-morpholino-3-fluoro-2-pyridylmethylthio)-(1H)-benzimidazole

(i) Carbon disulphide (3.8 ml) was added dropwise to a stirred solution of potassium hydroxide (1.8 g) in ethanol (50 ml) causing precipitation of the xanthate salt. 4,5-Dimethoxy-2,3-diaminobenzene (5 g) in ethanol (30 ml) was added dropwise to the stirred xanthate suspension at room temperature and heated under reflux for two hours. After the reflux, acetic acid (2.5 ml) in water (10 ml) was added dropwise and then excess solvent removed from the suspension. The resulting solid was washed with water and dried to give 5,6-dimethoxy-(1H)-benzimidazole-2-thiol (6.05 g) m.p. 278-280°.

(ii) Substituting 5,6-dimethoxy-(1H)-benzimidazole-2-thiol (1.6 g) for 5-methoxy-(1H)-benzimidazole-2-thiol and using corresponding molar proportions of the other reagents in the method of Example 1(vii), gave 5,6-dimethoxy-2-(4-morpholino-3-fluoro-2-pyridylmethylthio)-(1H)-benzimdazole (1.37 g) m.p. 173-174°.

Example 12

Preparation of 5,6-dimethoxy-2-(4-morpholino-3-fluoro-2-pyridylmethylsulphinyl)-(1H)-benzimidazole

(i) Substituting 5,6-dimethoxy-2-(4-morpholino-3-fluoro-2-pyridylmethylthio)-(1H)-benzimidazole (1.2 g) for 5-methoxy-2-(4-morpholino-3-fluoro-2-pyridylmethylthio)-(1H)-benzimidazole and using corresponding molar proportions of the other reagents in the method of Example 2, gave 5,6-dimethoxy-2-(4-morpholino-3-fluoro-2-pyridylmethylsulphinyl)-(1H)-benzimidazole (1.04 g) m.p. 213-215°.

$C_{19}H_{21}FN_4O_4S$

Found C,54.17 H,5.03 N,13.20 S,7.28

Requires C,54.27 H,5.03 N,13.33 S,7.63

Example 13

Preparation of 5-methyl-6-methoxy-2-(4-morpholino-3-fluoro-2-pyridylmethylthio)-(1H)-benzimidazole

(i) Substituting 4,5-diamino-2-methyl anisole (1.32 g) for 4,5-dimethoxy-2,3-diaminobenzene and using corresponding molar proportions of the other reagents in the method of Example 11, gave 5-methyl-6-methoxy-(1H)-benzimidazole-2-thiol (1.41 g) m.p. >310°.

(ii) Substituting 5-methyl-6-methoxy-(1H)-benzimidazole-2-thiol (1.9 g) for 5,6-dimethoxy-(1H)-benzimidazole-2-thiol and using corresponding molar proportions of the other reagents in the method of Example 11, gave 5-methyl-6-methoxy-2-(4-morpholino-3-fluoro-2-pyridylmethylthio)-(1H)-benzimidazole (1.6 g) m.p. 124-126°.

Example 14

Preparation of 5-methyl-6-methoxy-2-(4-morpholino-3-fluoro-2-pyridylmethylsulphinyl)-(1H)-benzimidazole

(i) Substituting 5-methyl-6-methoxy-2-(4-morpholino-3-fluoro-2-pyridylmethylthio)-(1H)-benzimidazole (1.1 g) for 5,6-dimethoxy-2-(4-morpholino-3-fluoro-2- pyridylmethylthio)-(1H)-benzimidazole and using corresponding molar proportions of the other reagents in the method of Example 2, gave 5-methyl-6-methoxy-2-(4-morpholino-3-fluoro-2-pyridylmethylsulphinyl)-(1H)-benzimidazole (0.85 g) m.p. 185-186°.

$C_{19}H_{21}FN_4O_3S$

Found C,56.54 H,5.27 N,13.69

Requires C,56.42 H,5.23 N,13.85

Example 15

Preparation of 5,6-dimethoxy-2-(4-dimethylamino-3-fluoro-2-pyridylmethylthio)-(1H)-benzimidazole

(i) Substituting 5,6-dimethoxy-(1H)-benzimidazole-2-thiol (1.87 g) for 5-methoxy-(1H)-benzimidazole-2-thiol and using corresponding molar proportions of the other reagents in the method of Example 5 (iii), gave 5,6-dimethoxy-2-(4-dimethylamino-3-fluoro-2-pyridylmethylthio)-(1H)-benzimidazole (1.53 g) m.p. 142-144°.

Example 16

Preparation of 5,6-dimethoxy-2-(4-dimethylamino-3-fluoro-2-pyridylmethylsulphinyl)-(1H)-benzimidazole

(i) Substituting 5,6-dimethoxy-2-(4-dimethylamino-3-fluoro-2-pyridylmethylthio)-(1H)-benzimidazole (1.5 g) for 5-methoxy-2-(4-dimethylamino-3-fluoro-2-pyridylmethylthio)-(1H)-benzimidazole and using corresponding molar proportions of the other reagents in the method of

Example 6, gave 5,6-dimethoxy-2-(4-dimethylamino-3-fluoro-2-pyridylmethylsulphinyl)-(1H)-benzimidazole (0.46 g) m.p. 208-210°.

$C_{17}H_{19}FN_4O_3S$

Found C 53.69, H 5.14, N 14.67

Requires C 53.96, H 5.06, N 14.81

## Example A

A tablet for oral administration is prepared by combining

| | Mg/Tablet |
|---|---|
| Compound of structure (I) | 100 |
| Mannitol | 153 |
| Starch | 33 |
| crospovidone | 12 |
| microcrystalline cellulose | 30 |
| magnesium stearate | 2 |
| | 330 mg |

into a 9 mm tablet. If the active ingredient is a compound of structure (I) in which n is 1 then the tablet is provided with an enteric coating.

## Example B

A pellet formulation for oral administration may be prepared by formulating the following into pellets by standard techniques

| | %w:w |
|---|---|
| Compound of structure (I) | 80 |
| microcrystalline cellulose | 10 |
| sodium carboxymethylcellulose | 2 |
| lactose | 8 |

If the active ingredient is a compound of structure (I) in which n is 1, the pellets are first enteric coated before being filled into hard gelatin capsules.

Example C

An injection for parenteral administration is prepared by combining

| | %w:w |
|---|---|
| Compound of Structure 1 | 1-5 |
| polypropylene glycol | 40 |
| ethanol | 10 |
| water for injection EP | to 100 |

The solution is then sterilised and sealed into 2 ml and 5 ml ampoules and vials.

Example D

A reconstitutable lyophilisate for parenteral administration is prepared from:

| | %w:w |
|---|---|
| Compound of structure (I) as a salt | 1-5 |
| Mannitol | 15 |
| NaCl | sufficient to make reconstituted solution isotonic |
| water | to 100 |

The solution is sterilised by aseptic filtration, 5 ml portions dispensed into 15 ml vials and the solution lyophilised. The lyophilisate can be reconstituted with 10 ml $H_2O$.

Biological Data

A. Rat : Lumen perfused stomach (histamine stimulated gastric acid secretion).

Using a modification of the procedure described by Ghosh and Schild (Br. J. Pharmacology, 13, 54, 1958), $ED_{50}$ values after intravenous administration were obtained as follows:

| Example | $ED_{50}$ µmol/kg or % inhibition at µ/kg |
|---|---|
| 2 | 0.7 |
| 4 | 51% |
| 6 | 26% |
| 8 | 37% |
| 12 | 33% |
| 14 | 1.51 |
| 16 | 46% |

B. Dog : Conscious Heidenhain Pouch (histamine-stimulated gastric acid secretion).

Using dogs surgically prepared with Heidenhain pouches 1 to 3 years previously, inhibitory $ED_{50}$ values against histamine stimulated gastric acid secretion after intravenous administration were obtained.

| Example | $ED_{50}$ µm/kg |
|---|---|
| 2 | 2.75 |
| 4 | 5.07 |
| 10 | 2.05 |
| 14 | 2.14 |

No overt signs of toxicity were observed in any of the foregoing tests.

Claims for Contracting States : BE, CH, DE, FR, GB, IT, LI, LU, NL and SE.

1. A compound of structure (I)

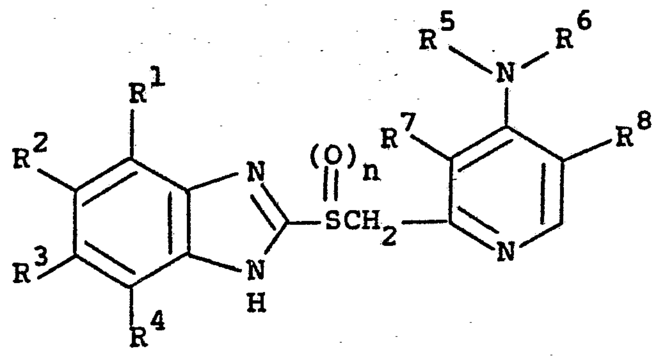

(I)

in which,

$R^1$ to $R^4$ are the same or different and are each hydrogen, halogen, trifluoromethyl, $C_{1-6}$alkyl, $C_{1-6}$alkoxy, $C_{1-6}$alkanoyl or $C_{1-6}$alkoxycarbonyl, $RCF_2O$, an ethoxy group substituted by 3 to 5 fluorine atoms, or

1$R^2$ and $R^3$ together form a group $-O(CR_2)_mO-$;

R is hydrogen or fluorine;

m is 1 or 2;

n is 0 or 1;

$R^5$ and $R^6$ are the same or different and are each hydrogen, $C_{1-6}$alkyl or $C_{3-6}$cycloalkyl, or $R^5$ and $R^6$ together with the nitrogen atom to which they are attached form an azetidino, pyrrolidino, piperidino, piperazino, N-$C_{1-4}$alkyl- piperazino or morpholino- group; and

one of $R^7$ and $R^8$ is fluorine, and the other is hydrogen, fluorine or $C_{1-6}$alkyl;

or a pharmaceutically acceptable salt thereof.

2. A compound as claimed in claim 1 in which $R^5$ and $R^6$ together with the nitrogen atom to which they are attached form a pyrrolidino, piperidino, piperazino, N-$C_{1-4}$alkylpiperazino or morpholino group.

3. A compound as claimed in claim 1 or claim 2 in which n is 1.

4. A compound as claimed in claim 1 which is:-

5-methoxy-2-(4-dimethylamino-5-fluoro-2-pyridylmethyl-sulphinyl)-(1H)-benzimidazole

5-methoxy-2-(4-dimethylamino-3-fluoro-2-pyridylmethyl-sulphinyl)-(1H)-benzimidazole

5-methoxy-2-(4-morpholino-3-fluoro-2-pyridylmethyl-sulphinyl)-(1H)-benzimidazole

4,5-dimethoxy-2-(4-morpholino-3-fluoro-2-pyridylmethyl-sulphinyl)-(1H)-benzimidazole

4,5-dimethoxy-2-(4-dimethylamino-3-fluoro-2-pyridylmethyl-sulphinyl)-(1H)-benzimidazole

5-methoxy-2-(4-morpholino-5-fluoro-2-pyridylmethyl-sulphinyl)-(1H)-benzimidazole

5-methoxy-2-(4-morpholino-3-methyl-5-fluoro-2-pyridylmethyl-sulphinyl)-(1H)-benzimidazole

5-methyl-6-methoxy-2-(4-morpholino-3-fluoro-2-pyridylmethyl-sulphinyl)-(1H)-benzimidazole

5. A pharmaceutical composition comprising a compound as claimed in claim 1 in association with a pharmaceutically acceptable carrier.

6. A pharmaceutical composition as claimed in claim 5, in which n is 1.

7. A pharmaceutical composition as claimed in claim 6, in a form suitable for parenteral administration.

8. A compound as claimed in any one of claims 1 to 4 for use as a therapeutic agent.

9. A compound as claimed in any one of claims 1 to 4 for use in the treatment of gastro-intestinal diseases caused or exacerbated by gastric acidity.

10. A process for the preparation of a compound as claimed in claim 1, which comprises

a) reacting a compound of structure (II)

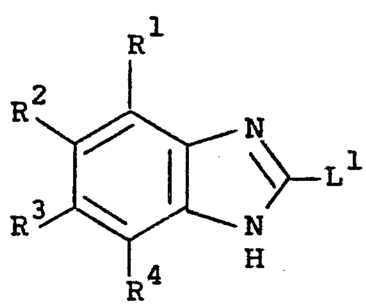

(II)

with a compound of structure (III)

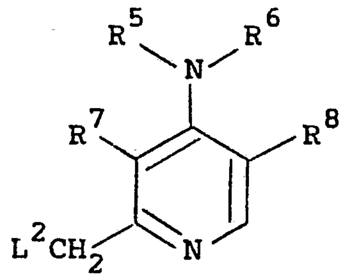

(III)

in which $R^1$ to $R^8$ are as described for structure (I), one of $L^1$ and $L^2$ is SH and the other is a leaving group displaceable by a mercaptan;

(b) reacting a compound of structure (IV)

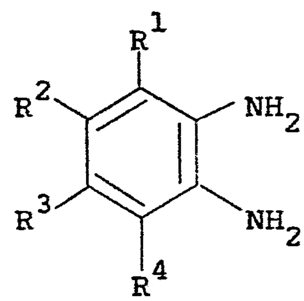

(IV)

in which $R^1$ to $R^4$ are as described for structure (I), with a compound of structure (V)

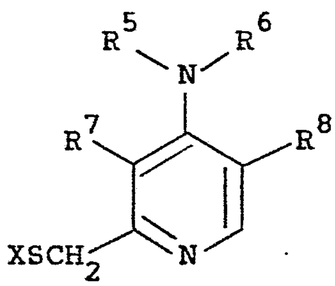

(V)

in which $R^5$ to $R^8$ are as described for structure (I), X is $CO_2H$ or $CSX^1$ and $X^1$ is halogen or $C_{1-4}$alkoxy;

(c) reacting a compound of structure (VI)

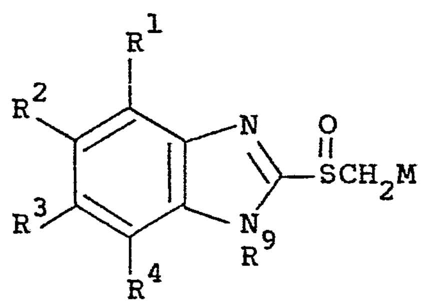

(VI)

in which $R^1$ to $R^4$ are as described for structure (I), $R^9$ is hydrogen or a protecting group and M is an alkali metal atom, with a compound of structure (VII)

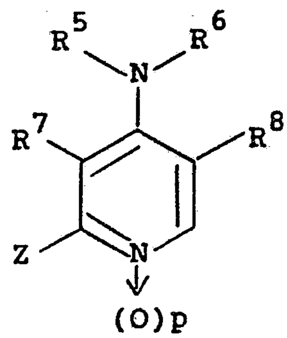

(VII)

in which $R^5$ to $R^8$ are as described for structure (I), Z is a leaving group and p is 0 or 1;

(d) reacting a compound of structure (VIII)

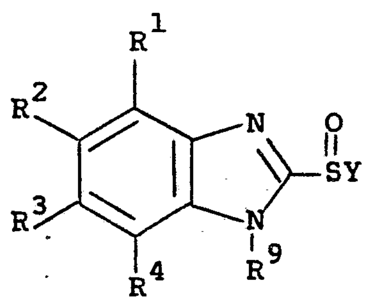

(VIII)

in which $R^1$ to $R^4$ are as described for structure (I), $R^9$ is hydrogen or a protecting group and Y is a leaving group, with a compound of structure (IX)

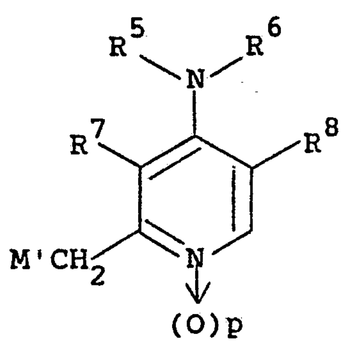

(IX)

in which $R^5$ to $R^8$ are as described for structure (I), p is 0 or 1 and M' is the equivalent of a metal atom,

and, optionally where desired,

(i) oxidising a compound of structure (I) in which n is 0 so formed to a compound of structure (I) in which n is 1;

(ii) reducing a compound of structure (I) so formed in which n is 1 to a compound of structure (I) in which n is 0;

(iii) forming a pharmaceutically acceptable salt.

11. A compound of structure (III)

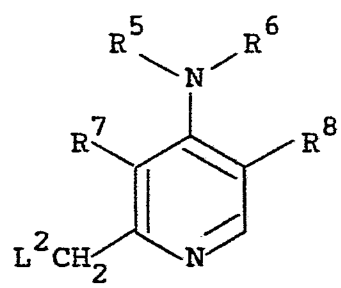

(III)

in which $R^5$ to $R^8$ are as described for structure (I), in claim 1 and $L^2$ is SH or a group displaceable by a mercaptan.

12. A compound of structure (V)

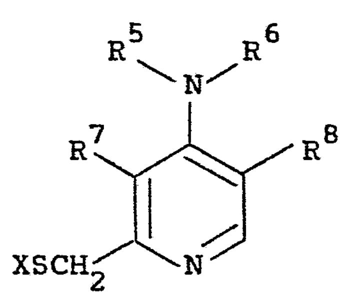

(V)

in which $R^5$ to $R^8$ are as described for structure (I), in claim 1 and X is $CO_2H$ or $CSX^1$ and $X^1$ is halogen or $C_{1-4}$alkoxy.

Claims for Contracting States : AT, GR and ES.

1. A process for the preparation of a compound of structure (I)

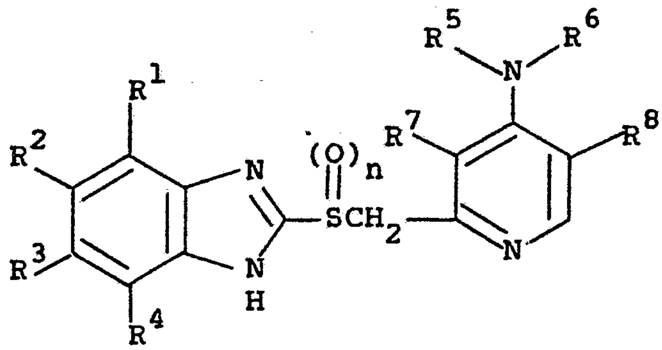

(I)

in which,

$R^1$ to $R^4$ are the same or different and are each hydrogen, halogen, trifluoromethyl, $C_{1-6}$alkyl, $C_{1-6}$alkoxy, $C_{1-6}$alkanoyl or $C_{1-6}$alkoxycarbonyl, $RCF_2O$, an ethoxy group substituted by 3 to 5 fluorine atoms, or $R^2$ and $R^3$ together form a group $-O(CR_2)_mO-$;

R is hydrogen or fluorine;

m is 1 or 2;

n is 0 or 1;

$R^5$ and $R^6$ are the same or different and are each hydrogen, $C_{1-6}$alkyl or $C_{3-6}$cycloalkyl, or $R^5$ and $R^6$ together with the nitrogen atom to which they are attached form an azetidino, pyrrolidino, piperidino, piperazino, N-$C_{1-4}$alkyl- piperazino or morpholino- group; and

one of $R^7$ and $R^8$ is fluorine, and the other is hydrogen, fluorine or $C_{1-6}$alkyl;

or a pharmaceutically acceptable salt thereof which comprises :

a) reacting a compound of structure (II)

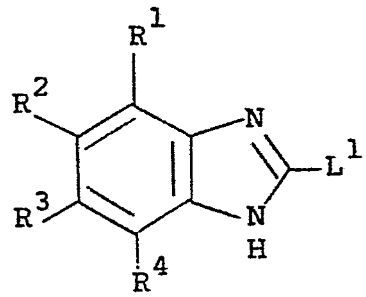

(II)

with a compound of structure (III)

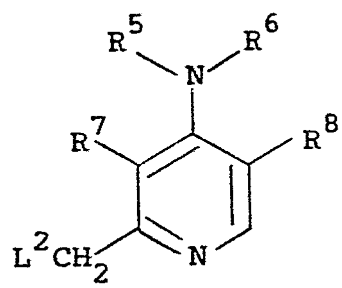

(III)

in which $R^1$ to $R^8$ are as described for structure (I), one of $L^1$ and $L^2$ is SH and the other is a leaving group displaceable by a mercaptan;

(b) reacting a compound of structure (IV)

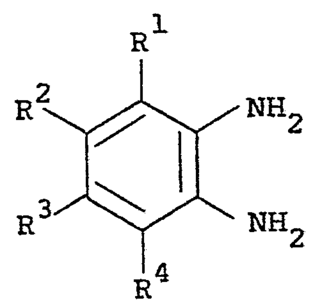

(IV)

in which $R^1$ to $R^4$ are as described for structure (I), with a compound of structure (V)

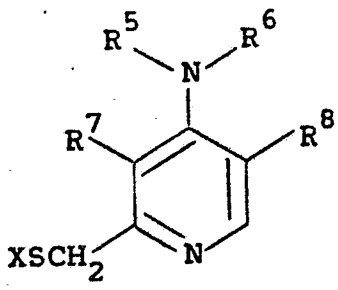

(V)

in which $R^5$ to $R^8$ are as described for structure (I), X is $CO_2H$ or $CSX^1$ and $X^1$ is halogen or $C_{1-4}$alkoxy;

(c) reacting a compound of structure (VI)

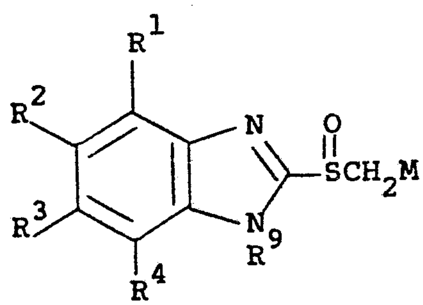

(VI)

in which $R^1$ to $R^4$ are as described for structure (I), $R^9$ is hydrogen or a protecting group and M is an alkali metal atom, with a compound of structure (VII)

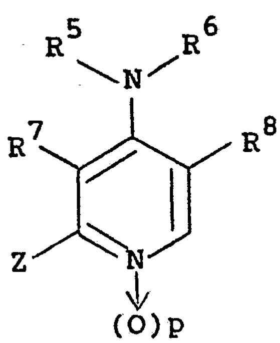

(VII)

in which $R^5$ to $R^8$ are as described for structure (I), Z is a leaving group and p is 0 or 1;

(d) reacting a compound of structure (VIII)

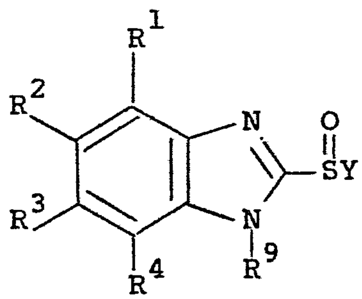

(VIII)

in which $R^1$ to $R^4$ are as described for structure (I), $R^9$ is hydrogen or a protecting group and Y is a leaving group, with a compound of structure (IX)

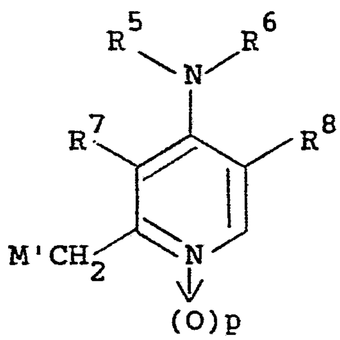

(IX)

in which $R^5$ to $R^8$ are as described for structure (I), p is O or 1 and M' is the equivalent of a metal atom,

and, optionally where desired,

(i) oxidising a compound of structure (I) in which n is O so formed to a compound of structure (I) in which n is 1;

(ii) reducing a compound of structure (I) so formed in which n is 1 to a compound of structure (I) in which n is O;

(iii) forming a pharmaceutically acceptable salt.

2. A process according to claim 1 in which $L^1$ is SH AND $L^2$ is a leaving group.

3. A process according to claim 2 in which $L^2$ is chlorine.

4. A process according to any one of claims 1 to 3 when used to prepare a compound of structure (I) in which n is 1.

5. A process according to any one of claims 1 to 4 when used to prepare a compound of structure (1) in which $R^5$ and $R^6$ together with the nitrogen atom to which they are attached form a pyrrolidino, piperidino, piperazino, $NC_{1-4}$alkyl piperazino or morpholino group.

6. A process according to any one of claims 1 to 4 when used to prepare a compound of structure (I) in which $R^5$ and $R^6$ are both $C_{1-6}$alkyl.

7. A process as claimed in any one of claims 1 to 3 when used to prepare

5-methoxy-2-(4-dimethylamino-5-fluoro-2-pyridylmethyl-sulphinyl)-(1H)-benzimidazole

5-methoxy-2-(4-dimethylamino-3-fluoro-2-pyridylmethyl-sulphinyl)-(1H)-benzimidazole

5-methoxy-2-(4-morpholino-3-fluoro-2-pyridylmethyl-sulphinyl)-(1H)-benzimidazole

4,5-dimethoxy-2-(4-morpholino-3-fluoro-2-pyridylmethyl-sulphinyl)-(1H)-benzimidazole

4,5-dimethoxy-2-(4-dimethylamino-3-fluoro-2-pyridylmethyl-sulphinyl)-(1H)-benzimidazole

5-methoxy-2-(4-morpholino-5-fluoro-2-pyridylmethyl-sulphinyl)-(1H)-benzimidazole

5-methoxy-2-(4-morpholino-3-methyl-5-fluoro-2-pyridylmethyl-sulphinyl)-(1H)-benzimidazole

5-methyl-6-methoxy-2-(4-morpholino-3-fluoro-2-pyridylmethyl-sulphinyl)-(1H)-benzimidazole

8. A process for preparing a pharmaceutical composition which comprises bringing into association a compound of structure (I) according to claim 1 and a pharmaceutically acceptable carrier.

9. A process according to claim 8 in which, in the compound of structure (I) n is 1.

10. A process according to claim 9 which comprises the further step of providing the composition with an enteric coating.

0246774

European Patent Office

# EUROPEAN SEARCH REPORT

Application number

EP 87 30 3915

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| X | EP-A-0 178 438 (BEECHAM)<br>* Pages 1-5,9,10,18-21 *<br>--- | 1-11 | C 07 D 401/12<br>C 07 D 491/04<br>A 61 K 31/415<br>A 61 K 31/44<br>C 07 D 213/73 |
| D,X | EP-A-0 184 322 (SKF)<br>* Column 1, formula I, lines 55,56; column 2, line 60, column 3, lines 14,15; column 5, formula III, lines 63-65; column 7, formula V, lines 32-34; columns 19-21 *<br>----- | 1-12 | |
| | | | TECHNICAL FIELDS SEARCHED (Int Cl 4)<br>C 07 D 401/00<br>C 07 D 491/00<br>A 61 K 31/00<br>C 07 D 213/00 |

The present search report has been drawn up for all claims

| Place of search | Date of completion of the search | Examiner |
|---|---|---|
| THE HAGUE | 07-08-1987 | DE BUYSER I.A.F. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document